# FASCICULE DE BREVET EUROPEEN

(11) **EP 1 873 140 B1**
(45) Date de publication et mention de la délivrance du brevet: **30.09.2009**
(21) Numéro de dépôt: 07290819.7
(22) Date de dépôt: 29.06.2007
(51) Int. Cl.: C07C 233/18, A61K 31/165, A61P 25/00, A61P 9/00, A61P 15/00, A61P 37/02, A61P 35/00

(54) **Nouveaux dérivés naphtaleniques, leur procédé de préparation et les compositions pharmaceutiques qui les contiennent**
Neue Naphtalinderivate, ihr Herstellungsverfahren und die pharmazeutischen Zusammensetzungen, die sie enthalten
New naphthalene derivatives, method of preparing same and pharmaceutical compositions containing them

(30) Priorité: 30.06.2006 FR 0605917
(43) Date de publication de la demande: 02.01.2008
(73) Titulaire: Les Laboratoires Servier, 92415 Courbevoie Cedex (FR)
(72) Inventeur: Yous, Said, 59120 Loos (FR); Peres, Basile, 09000 Foix (FR); Sabaouni, Ahmed, 59280 Armentieres (FR); Berthelot, Pascal, 59320 Haubourdin (FR); Spedding, Michael, 78110 Le Vesinet (FR); Delagrange, Philippe, 92130 Issy les Moulineaux (FR); Caignard, Daniel-Henri, 95000 Boisemont (FR); Millan, Mark, 78230 Le Pecq (FR)

(56) Documents cités:
- EP-A1- 0 562 956
- EP-A1- 0 994 102
- MAROT C ET AL: "PHARMACOPHORIC SEARCH AND 3D-QSAR COMPARATIVE MOLECULAR FIELD ANALYSIS STUDIES ON AGONISTS OF MELATONIN SHEEP RECEPTORS" JOURNAL OF MEDICINAL CHEMISTRY, AMERICAN CHEMICAL SOCIETY. WASHINGTON, US, vol. 41, no. 23, 1998, pages 4453-4465, XP002172225 ISSN: 0022-2623

## Description

La présente invention concerne de nouveaux dérivés naphtaléniques, leur procédé de préparation et les compositions pharmaceutiques qui les contiennent.

Les composés de la présente invention sont nouveaux et présentent des caractéristiques pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle des rythmes circadiens. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp. 321-341) et du sommeil (Psychopharmacology, 1990, 100, pp. 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp. 264-272), analgésiques (Pharmacopsychiat., 1987, 20, pp. 222-223), ainsi que pour le traitement des maladies de Parkinson (J. Neurosurg. 1985, 63, pp. 321-341) et d'Alzheimer (Brain Research, 1990, 528, pp. 170-174). De même, ces composés ont montré une activité sur certains cancers (Melatonin - Clinical Perspectives, Oxford University Press, 1988, pp. 164-165), sur l'ovulation (Science 1987, 227, pp. 714-720), sur le diabète (Clinical Endocrinology, 1986, 24, pp. 359-364), et dans le traitement de l'obésité (International Journal of Eating Disorders, 1996, 20 (4), pp. 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p. 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands sélectifs.

De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment. Des dérivés naphtaléniques ont été décrits en tant que modulateurs des récepteurs de la mélatonine dans Marot et al. (J. Med. Chem. 1998, 41, pp. 4453-4465) et dans la demande de brevet EP 0994102.

Les composés de la présente invention outre leur nouveauté, montrent une très forte affinité pour les récepteurs de la mélatonine.

Ils présentent par ailleurs une forte affinité pour le récepteur 5-HT_{2C}, ce qui a pour effet de renforcer les propriétés observées avec les récepteurs mélatoninergiques, notamment dans le domaine de la dépression.

La présente invention concerne plus particulièrement le composé de formule (I) : 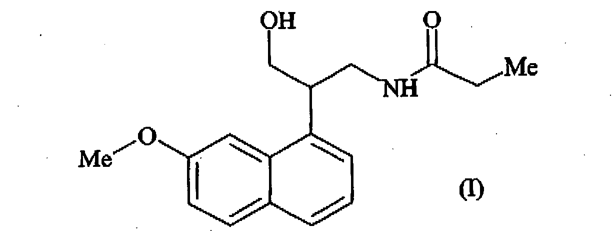 ses énantiomères, ainsi que ses sels d'addition à une base pharmaceutiquement acceptable.

Parmi les bases pharmaceutiquement acceptables, on peut citer à titre non limitatif l'hydroxyde de sodium, l'hydroxyde de potassium, la triéthylamine, la tertbutylamine, etc.

Encore plus particulièrement, l'invention concerne les composés qui sont le *N*-[3-hydroxy-2-(7-méthoxy-1-naphtyl)propyl]propanamide, le *N*-[(2*S*)-3-hydroxy-2-(7-méthoxy-1-naphtyl)propyl]propanamide, et le *N*-[(2*R*)-3-hydroxy-2-(7-méthoxy-1-naphtyl)propyl] propanamide.

Les sels d'addition à une base pharmaceutiquement acceptable des composés préférés de l'invention font partie intégrante de l'invention.

L'invention s'étend également au procédé de préparation du composé de formule (I) caractérisé en ce que l'on utilise comme produit de départ le composé de formule (II) : 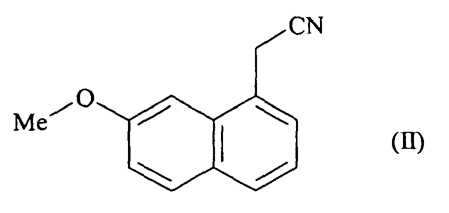 que l'on soumet en milieu basique à l'action du carbonate de diméthyle pour conduire au composé de formule (III) : 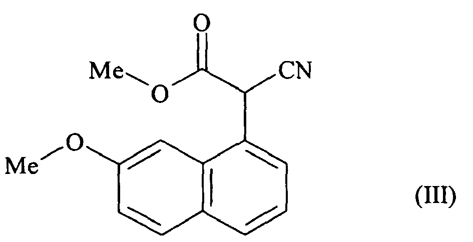 que l'on soumet à une réduction en présence d'un hydrure pour conduire au composé de formule (IV) : 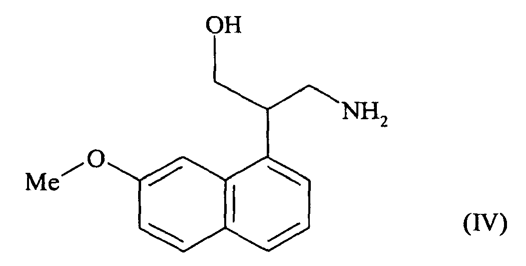 sur lequel on condense le chlorure de propanoyle pour conduire au composé de formule (I),
qui peut être purifié selon une technique classique de séparation, que l'on transforme, si on le souhaite, en ses sels d'addition à une base pharmaceutiquement acceptable et dont les énantiomères peuvent être séparés sur colonne chirale selon une technique classique de séparation.

L'étude pharmacologique des dérivés de l'invention a montré qu'ils étaient atoxiques, doués d'une forte affinité sélective pour les récepteurs de la mélatonine et possédaient d'importantes activités sur le système nerveux central et, en particulier, on a relevé des propriétés thérapeutiques sur les troubles du sommeil, des propriétés antidépressives, anxiolytiques, antipsychotiques, analgésiques ainsi que sur la microcirculation, qui permettent d'établir que les produits de l'invention sont utiles dans le traitement du stress, des troubles du sommeil, de l'anxiété, des dépressions saisonnières ou de la dépression majeure, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immunomodulateurs et qu'ils sont susceptibles d'être utilisés dans le traitement des cancers.

Les composés seront utilisés de préférence dans les traitements de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

Par exemple, les composés seront utilisés dans le traitement de la dépression majeure, des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Les exemples suivants illustrent l'invention et ne la limitent en aucune façon.

### Exemple 1 : N-[3-Hydroxy-2-(7-méthoxy-1-naphtyl)propyl]propanamide

### Stade A : Cyano(7-méthoxy-1-naphtyl)acétate de méthyle

Le (7-méthoxy-napht-1-yl)acétonitrile (20 g) est dissous dans 150 ml de tétrahydrofurane anhydre. L'hydrure de sodium (202,8 mmol) est ajouté à température ambiante, et le milieu est chauffé à reflux pendant 30 minutes. Le carbonate de diméthyle (12 ml) est ajouté avec précaution et le milieu réactionnel est chauffé à reflux pendant 30 minutes. Le milieu est versé dans l'eau glacée et la phase aqueuse est acidifiée avec 21 ml d'une solution d'acide chlorhydrique à 37 % puis extraite par 2 fois 100 ml d'éther. La phase organique est lavée à l'eau, séchée, décolorée et évaporée. L'huile obtenue précipite dans l'éther et le précipité formé est essoré puis recristallisé pour conduire au produit du titre sous la forme d'un solide blanc.
*Point de fusion : 80-82°C*

### Stade B: 3-Amino-2-(7-méthoxy-1-naphtyl)-1-propanol, chlorhydrate

Le chlorure d'aluminium (80 mmol), solubilisé dans 200 ml d'éther anhydre, est ajouté à une suspension d'hydrure mixte d'aluminium et de lithium à 0°C dans 300 ml d'éther anhydre. Après 10 minutes d'agitation le composé obtenu au Stade A (20 mmol) est ajouté en solution dans 200 ml d'éther anhydre. Après 30 minutes, le milieu est hydrolysé à froid et avec précaution avec une solution de soude (10 g ; 40 ml). Le précipité formé est ensuite filtré et abondamment lavé à l'éther. Le résidu obtenu après évaporation est repris par l'eau et la phase aqueuse est extraite au dichlorométhane. La phase organique est ensuite lavée à l'eau, séchée, décolorée, puis traitée par l'acide chlorhydrique gazeux et évaporée. L'huile obtenue précipite dans l'acétate d'éthyle et le précipité formé est essoré puis recristallisé pour conduire au produit du titre sous la forme d'un solide blanc.
*Point de fusion : 164-166°C*

### Stade C : N-[3-Hydroxy-2-(7-méthoxy-1-naphtyl)propyl]propanamide

Le composé obtenu au Stade B (3,73 mmol) est mis en solution dans 100 ml d'un mélange eau-acétate d'éthyle (50/50). Le carbonate de potassium (11,2 mmol) est ajouté et le milieu réactionnel est refroidi à 0°C avec un bain de glace. Le chlorure de propanoyle (4,6 mmol) est ajouté goutte à goutte et le milieu est agité pendant 15 minutes à froid. En fin de réaction, la phase organique est lavée avec une solution d'acide chlorhydrique (1M), lavée à l'eau, séchée et évaporée sous pression réduite. Le solide obtenu est recristallisé dans de l'acétonitrile pour conduire au produit du titre sous la forme d'un solide blanc.
*Point de fusion : 128-129°C*

| *Microanalyse élémentaire :* | | | | |
|---|---|---|---|---|
| | % | *C* | *H* | *N* |
| *Calculé :* | | *71,05* | *7,36* | *4,77* |
| *Trouvé :* | | *70,82* | *7,39* | *4,70* |

Le composé obtenu dans l'Exemple 1 est purifié sur colonne chirale (R,R)WHELK 0-1 avec un éluant toluène/n-propanol (100/25) et une détection à 340 nm, pour conduire aux Exemples 2 et 3 :

### Exemple 2 : N-[(2S)-3-Hydroxy-2-(7-méthoxy-1-naphtyl)propyl]propanamide

### Exemple 3 : N-[(2R)-3-Hydroxy-2-(7-méthoxy-1-naphtyl)propyl]propanamide

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Test de la nage forcée

Les composés de l'invention sont testés dans un modèle comportemental, le test de la nage forcée.

L'appareil est constitué d'un cylindre en plexiglas rempli d'eau. Les animaux sont testés individuellement pendant une session de 6 minutes. Au début de chaque test, l'animal est placé au centre du cylindre. Le temps d'immobilisation est enregistré. Chaque animal est jugé immobile quand il cesse de se débattre, et reste à la surface de l'eau, immobile, ne faisant seulement que les mouvements lui permettant de maintenir la tête hors de l'eau.

Après administration 40 minutes avant le début du test, les composés de l'invention diminuent de façon significative la durée d'immobilisation attestant de leur activité antidépressive.

### EXEMPLE C : Etude de liaison aux récepteurs MT₁ et MT₂ de la mélatonine

Les expériences de liaison aux récepteurs MT₁ ou MT₂ sont réalisées en utilisant la 2-[¹²⁵I]-iodomélatonine comme radioligand de référence. La radioactivité retenue est déterminée à l'aide d'un compteur à scintillation liquide.
Des expériences de liaison compétitive sont ensuite réalisées en triple, avec les différents composés à tester. Une gamme de concentrations différentes est testée pour chaque composé. Les résultats permettent de déterminer les affinités de liaison des composés testés (Kᵢ).

A titre d'exemple, le composé obtenu dans l'Exemple 1 présente un Kᵢ(MT₁) de 1,4 nM, et un Kᵢ(MT₂) de 3,2 nM.

### EXEMPLE D : Etude de la liaison aux récepteurs sérotoninergiques 5-HT_{2C}

L'affinité des composés pour le récepteur humain 5-HT_{2C} est évaluée sur des préparations membranaires de cellules CHO exprimant de façon stable ce récepteur.
L'incubation est réalisée dans du tampon TRIS 50 mM, pH 7,4 contenant du MgCl₂ 10 mM et de la BSA 0,1%, en présence de [³H]Mésulergine (1 nM) et de 25 fmol/ml de récepteur. La liaison non-spécifique est déterminée en présence de miansérine 10µM.
La réaction est stoppée par l'ajout de tampon TRIS 50 mM, pH 7,4 suivi d'une étape de filtration et de 3 rinçages successifs : la radioactivité liée aux membranes restant sur les filtres (GF/B prétraités au PEI 0.1 %) est comptée en scintillation liquide.

Les résultats obtenus montrent que les composés de l'invention sont affins pour le récepteur 5-HT_{2C} avec des Kᵢ < 10 µM.

### EXEMPLE E : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats

Il apparaît clairement que les composés de l'invention permettent d'agir de façon puissante sur le rythme circadien *via* le système mélatoninergique.

### EXEMPLE F : Test des cages claires/obscures

Les composés de l'invention sont testés dans un modèle comportemental, le test des cages claires/obscures, qui permet de révéler l'activité anxiolytique des molécules.

L'appareil est composé de deux boîtes en polyvinyle couvertes de Plexiglas. L'une de ces boîtes est obscure. Une lampe est placée au-dessus de l'autre boîte donnant une intensité lumineuse au centre de celle-ci d'approximativement 4000 lux. Un tunnel opaque en plastique sépare la boîte claire de la boîte sombre. Les animaux sont testés individuellement pendant une session de 5 min. Le plancher de chaque boîte est nettoyé entre chaque session. Au début de chaque test, la souris est placée dans le tunnel, face à la boîte sombre. Le temps passé par la souris dans la boîte éclairée et le nombre de transitions à travers le tunnel sont enregistrés après la première entrée dans la boîte sombre.

Après administration des composés 30 min avant le début du test, les composés de l'invention augmentent de façon significative le temps passé dans la cage éclairée ainsi que le nombre des transitions, ce qui montre l'activité anxiolytique des dérivés de l'invention.

### EXEMPLE G : Composition pharmaceutique : Comprimés

| | |
|---|---|
| 1000 comprimés dosés à 5 mg de *N*-[3-hydroxy-2-(7-méthoxy-1-naphtyl)propyl] propanamide (Exemple 1) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composé de formule (I) : 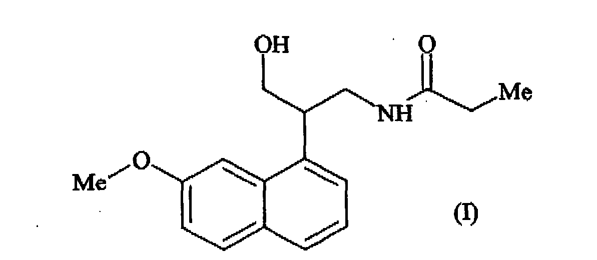 ou N-[3-hydroxy-2-(7-méthoxy-1-naphtyl)propyl]propanamide
ses énantiomères, ainsi que ses sels d'addition à une base pharmaceutiquement acceptable.

2. Composé de formule (I) selon la revendication 1 qui est le *N*-[(2S)-3-hydroxy-2-(7-méthoxy-1-naphtyl)propyl] propanamide, ainsi que ses sels d'addition à une base pharmaceutiquement acceptable.

3. Composé de formule (I) selon la revendication 1 qui est le *N*-[(2R)-3-hydroxy-2-(7-méthoxy-1-naphtyl)propyl] propanamide, ainsi que ses sels d'addition à une base pharmaceutiquement acceptable.

4. Procédé de préparation du composé de formule (I) selon la revendication 1 **caractérisé en ce que** l'on utilise comme produit de départ le composé de formule (II) : 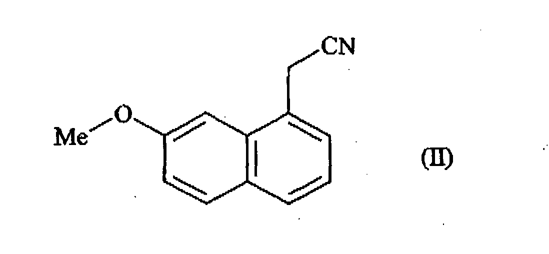 que l'on soumet en milieu basique à l'action du carbonate de diméthyle pour conduire au composé de formule (III) : 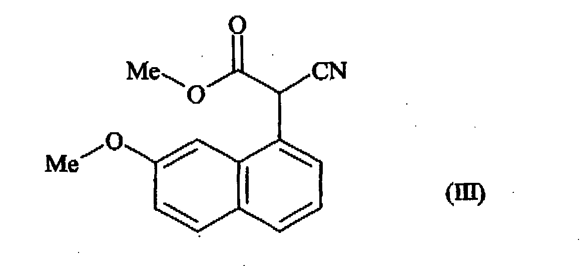 que l'on soumet à une réduction en présence d'un hydrure pour conduire au composé de formule (IV) : 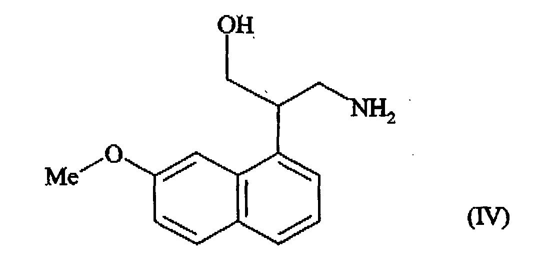 sur lequel on condense le chlorure de propanoyle pour conduire au composé de formule (I) qui peut être purifié selon une technique classique de séparation, que l'on transforme, si on le souhaite, en ses sels d'addition à une base pharmaceutiquement acceptable et dont les énantiomères peuvent être séparés sur colonne chirale selon une technique classique de séparation.

5. Compositions pharmaceutiques contenant au moins un composé de formule (I) selon l'une quelconque des revendications 1 à 3 ou un de leurs sels d'addition avec une base pharmaceutiquement acceptable en combinaison avec un ou plusieurs excipients pharmaceutiquement acceptables.

6. Compositions pharmaceutiques selon la revendication 5 utiles pour la fabrication de médicaments pour traiter les troubles du système mélatoninergique.

7. Compositions pharmaceutiques selon la revendication 5 utiles pour la fabrication de médicaments pour le traitement des troubles du sommeil, du stress, de l'anxiété, de la dépression majeure ou des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, des troubles de la circulation cérébrale, ainsi que dans les dysfonctionnements sexuels, en tant qu'inhibiteurs de l'ovulation, d'immunomodulateurs et dans le traitement des cancers.

## Claims

1. Compound of formula (I) : 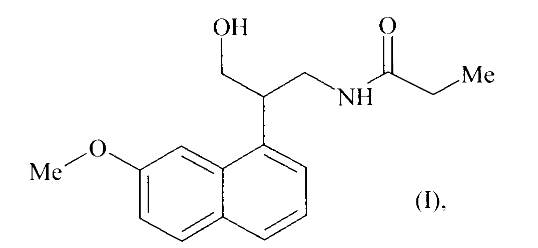 or N-[3-hydroxy-2-(7-methoxy-1-naphthyl)propyl]propanamide,
enantiomers thereof, and addition salts thereof with a pharmaceutically acceptable base.

2. Compound of formula (I) according to claim 1, which is *N*-[(2S)-3-hydroxy-2-(7-methoxy-1-naphthyl)propyl]propanamide, and addition salts thereof with a pharmaceutically acceptable base.

3. Compound of formula (I) according to claim 1, which is *N*-[(2R)-3-hydroxy-2-(7-methoxy-1-naphthyl)propyl]propanamide, and addition salts thereof with a pharmaceutically acceptable base.

4. Process for the preparation of the compound of formula (I) according to claim 1, **characterised in that** there is used as starting material the compound of formula (II) : 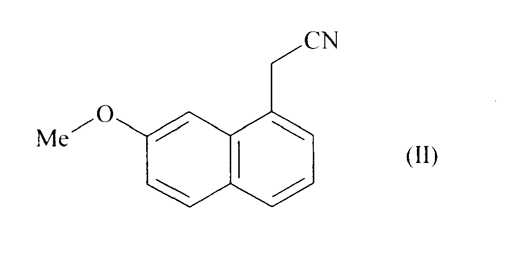 which is subjected to the action of dimethyl carbonate in a basic medium to yield the compound of formula (III) : 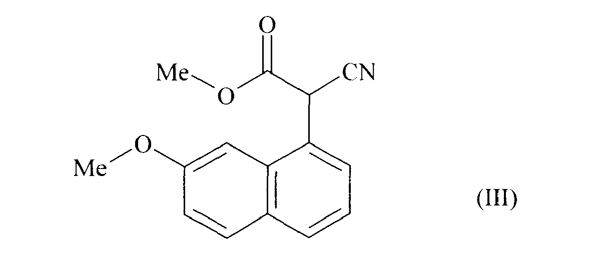 which is subjected to reduction in the presence of a hydride to yield the compound of formula (IV) : 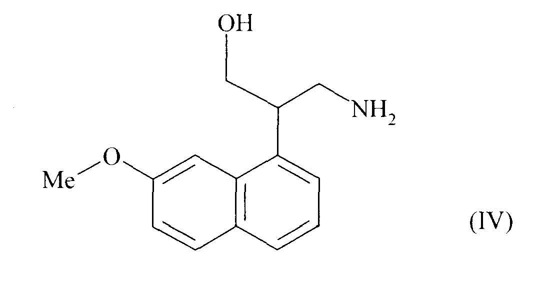 with which propanoyl chloride is condensed to yield the compound of formula (I), which may be purified according to a conventional separation technique, which is converted, if desired, into its addition salts with a pharmaceutically acceptable base and the enantiomers of which may be separated on a chiral column according to a conventional separation technique.

5. Pharmaceutical compositions comprising at least one compound of formula (I) according to any one of claims 1 to 3 or an addition salt thereof with a pharmaceutically acceptable base in combination with one or more pharmaceutically acceptable excipients.

6. Pharmaceutical compositions according to claim 5 for use in the manufacture of medicaments for treating disorders of the melatoninergic system.

7. Pharmaceutical compositions according to claim 5 for use in the manufacture of medicaments for the treatment of sleep disorders, stress, anxiety, major depression or seasonal affective disorder, cardiovascular pathologies, pathologies of the digestive system, insomnia and fatigue due to jetlag, schizophrenia, panic attacks, melancholia, appetite disorders, obesity, insomnia, psychotic disorders, epilepsy, diabetes, Parkinson's disease, senile dementia, various disorders associated with normal or pathological ageing, migraine, memory loss, Alzheimer's disease, cerebral circulation disorders or sexual dysfunctions, as ovulation-inhibitors or immunomodulators, or for the treatment of cancers.

## Patentansprüche

1. Verbindung der Formel (I): 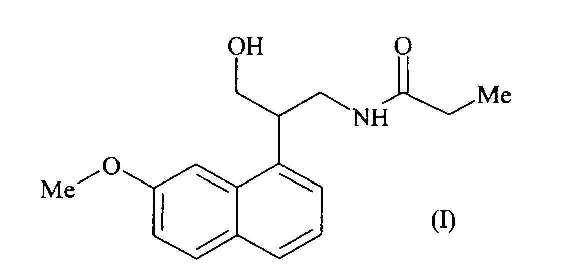 oder N-[3-Hydroxy-2-(7-methoxy-1-naphthyl)-propyl]-propanamid,
seine Enantiomeren sowie seine Additionssalze mit einer pharmazeutisch annehmbaren Base.

2. Verbindung der Formel (I) nach Anspruch 1, nämlich *N*-[(2S)-3-Hydroxy-2-(7-methoxy-1-naphthyl)-propyl]-propanamid sowie seine Additionssalze mit einer pharmazeutisch annehmbaren Base.

3. Verbindung der Formel (I) nach Anspruch 1, nämlich *N*-[(2R)-3-Hydroxy-2-(7-methoxy-1-naphthyl)-propyl]-propanamid sowie seine Additionssalze mit einer pharmazeutisch annehmbaren Base.

4. Verfahren zur Herstellung der Verbindung der Formel (I) nach Anspruch 1, **dadurch gekennzeichnet, dass** man als Ausgangsmaterial die Verbindung der Formel (II) verwendet: 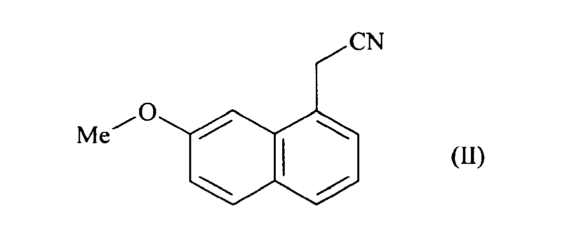 welche man in basischem Medium der Einwirkung von Dimethylcarbonat unterwirft zur Bildung der Verbindung der Formel (III): 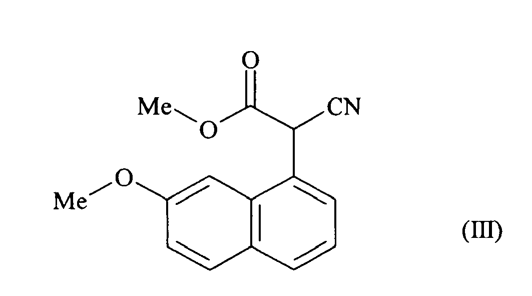 welche man einer Reduktion in Gegenwart eines Hydrids unterzieht zur Bildung der Verbindung der Formel (IV): 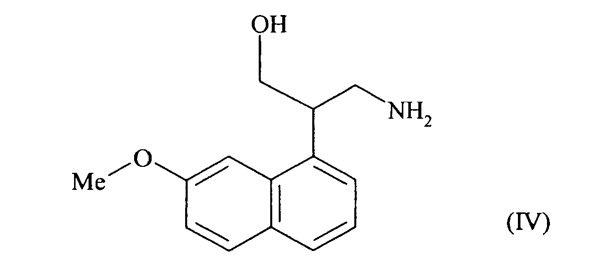 welche man mit Propanoylchlorid kondensiert zur Bildung der Verbindung der Formel (I), welche mit Hilfe einer klassischen Trennmethode gereinigt werden kann, welche man gewünschtenfalls in Säureadditionssalze mit einer pharmazeutisch annehmbaren Base überführt und dessen Enantiomeren mit Hilfe einer klassischen Trennmethode auf einer chiralen Säule getrennt werden können.

5. Pharmazeutische Zubereitungen enthaltend mindestens eine Verbindung der Formel (I) nach einem der Ansprüche 1 bis 3 oder eines ihrer Additionssalze mit einer pharmazeutisch annehmbaren Base in Kombination mit einem oder mehreren pharmazeutisch annehmbaren Hilfsstoffen.

6. Pharmazeutische Zubereitungen nach Anspruch 5, nützlich für die Herstellung von Arzneimitteln zur Behandlung von Störungen des melatoninergischen Systems.

7. Pharmazeutische Zubereitungen nach Anspruch 5, nützlich für die Herstellung von Arzneimitteln zur Behandlung von Schlafstörungen, Stress, Angst, starken Depressionen oder saisonal bedingten Depressionen, kardiovaskulären Erkrankungen, Erkrankungen des Verdauungssystems, Schlaflosigkeit und Müdigkeit als Folge von Zeitverschiebungen, der Schizophrenie, von Panikanfällen, der Melancholie, Appetitstörungen, Fettsucht, Schlaflosigkeit, von psychischen Störungen, der Epilepsie, des Diabetes, der Parkinsonschen Krankheit, der senilen Demenz, von verschiedenen Störungen, die mit dem normalen oder pathologischen Altern verknüpft sind, der Migräne, von Gedächtnisverlusten, der Alzheimerschen Krankheit, von Störungen der Gehirndurchblutung sowie bei sexuellen Dysfunktionen als Inhibitoren der Ovulation, Immunomodulatoren und bei der Behandlung von Krebs.
